# EUROPEAN PATENT SPECIFICATION

(11) **EP 1 019 029 B1**
(45) Date of publication and mention of the grant of the patent: **03.09.2003**
(21) Application number: 98950099.6
(22) Date of filing: 01.10.1998
(51) Int. Cl.: A61K 9/20

(54) **MONOLITHIC SYSTEM CONTAINING ONE OR MORE DRUGS, CONSISTING OF THREE LAYERS WITH DIFFERENT RELEASE MECHANISMS**
MONOLITHSYSTEME ENTHALTEND MINDESTENS EINER WIRKSTOFF, BESTEHEND AUS DREI SCHICHTEN MIT UNTERSCHIEDLICHEN FREISETZUNGMECHANISMUS
SYSTEME MONOLITHIQUE CONTENANT UN OU PLUSIEURS MEDICAMENTS CONSTITUE DE TROIS COUCHES A MECANISMES DE LIBERATION DIFFERENTS

(30) Priority: 03.10.1997 IT MI972254
(43) Date of publication of application: 19.07.2000
(73) Proprietor: CHIESI FARMACEUTICI S.p.A., I-43100 Parma (IT)
(72) Inventor: CHIESI, Paolo, I-43100 Parma (IT); VENTURA, Paolo, I-43100 Parma (IT); ACERBI, Daniela, I-43100 Parma (IT); MUSA, Rossela, I-43100 Parma (IT); BETTINI, Ruggero, I-43100 Parma (IT); CAPONETTI, Giovanni, I-43100 Parma (IT); CATELLANI, Pier, Luigi, I-43100 Parma (IT)
(74) Representative: Minoja, Fabrizio, Dr.
(86) International application number: EP9806228
(87) International publication number: WO99017745

(56) References cited:
- EP-A- 0 598 309
- EP-A- 0 788 790
- WO-A-94/06416
- WO-A-95/01781

## Description

The controlled release of drugs allows the revaluation of many active molecules, determining more stable blood levels of the active ingredient and, consequently, an improved therapeutic control with a reduction in side effects related to fluctuating and uncontrolled drug blood levels. Furthermore it allows to simplify the dosage schemes with a greater patient's compliance and adherence to the therapy which brings to a greater clinical efficacy.

One of the most frequently used route of administration of controlled drug release formulations is the oral route, for which monolithic systems obtained by compression are proposed. These systems often act as matrices with different release mechanism which can derive from erosion of the matrix or from its physical transformation due to the swelling of a hydrophilic component, usually a polymer. Other release forms consist of monolithic systems coated with a polymeric membrane, in which the release mechanism is determined by diffusion of the drug through the membrane.

Recently, many proposals for monolithic systems have appeared in the patent literature, characterized by the presence of different layers, each of which intended for the release of one or more active ingredients at different rates. For example, in U.S. patent 4839177, a controlled release system is described, consisting of a core containing the drug and a swelling polymer, of cylindrical shape, characterized by the presence of an insoluble coating partially applied on one or more of its faces.

The purpose of the coating is to modulate the swelling so as to control the release of the drug. In patent application WO 94/06416, a multi-layer tablet has been described, consisting of a first layer containing one or more drugs with immediate or controlled release formulation, a second layer containing one or more drugs with slow release formulation and a third layer with the function of a low permeability barrier, coating the second layer or alternatively placed between the first two layers. Also in patent application WO 95/01781, a multi-layer tablet is described, in which a first layer is intended to a fast release of a portion of the drug, a second layer and optionally a third one, is intended to release the drug gradually. Lastly, in patent EP 0 598 309 A2, a multi-layer tablet is described, for the controlled release of active ingredient, consisting of at least one swelling layer containing one or more active ingredients and of at least one soluble layer containing one or more active ingredients, in contact with the swelling layer.

From the analysis of the systems described in the above cited patents, it emerges that the release performance of each layer is independent on the relative position of the layers but depends entirely on their formulation. In no previous document the possibility of using the interaction between adjacent layers having different release mechanisms has been taken into consideration. In no previous document the possibility of producing, in a monolithic system, a total release kinetic of the active ingredient/s as a function of the relative positions of the layers other than as the sum of the individual contributions of each individual layer is disclosed.

Three well known mechanisms of drug release from pharmaceutical forms for oral administration are based on processes of disintegration, erosion and swelling.

In particular
- the disintegration mechanism allows rapid release of the drug which dissolves and is absorbed with a kinetic of the first order;
- the erosion mechanism allows gradual release of the drug with a kinetic near to constant release or of zero order;
- the swelling mechanism allows slow release of the drug with a kinetic defined as anomalous-Fickian.

It has now been found that, in a three-layer monolithic system, in which one layer is based on disintegration (layer D), one layer is based on erosion (layer E) and one layer is based on swelling (layer R), according to the release mechanism and to the relative position of each layer, the three mechanisms interact to give a whole drug release which substantially differs from the simple sum of the individual contributions. The drug release changes according to the release mechanism proper of a certain layer and depending on the relative position of the layers forming the monolithic system. According to a first object of the invention a controlled release form for oral administration is provided, consisting of a monolithic system comprising a disintegrating layer, an erodible layer and a swelling layer of which two external and one intermediate, each layer optionally containing one or more drugs.

In particular, according to a preferred embodiment of the invention, when the disintegrating layer (layer D) is interposed between the erodible layer (layer E) and the swelling layer (layer R), by contact with water, it rapidly disintegrates causing an immediate release of the drug(s) and at the same time the removal of the two external layers. As a consequence, the dissolution of the drug(s) contained in the external layers, by the effects of the erosion and swelling mechanism takes place in a slow and controlled manner.

The usefulness of the three-layer monolithic system is particularly evident in the case of contemporary administration of two active ingredients having different chemical, physical and pharmacokinetic properties. Levodopa and Carbidopa for example, are usually associated in the treatment of Parkinson's disease, since Carbidopa is a peripheral inhibitor of the enzymatic system involved in the metabolic transformation of Levodopa to dopamine. Levodopa methylester is a pro-drug of Levodopa, characterized by high water solubility and rapid absorption after oral administration. Levodopa methylester is rapidly absorbed and completely hydrolized to Levodopa already at a pre-systemic level. It is of therapeutic importance that the transformation of Levodopa to dopamine takes place at a cerebral rather than a peripheral level, since the activity of the biogenic amine in the Central Nervous System is desirable while it is undesirable at a peripheral level where it can produce side effects. The simultaneous and preferably sequential administration of the inhibitor prolongs the permanence in the systemic circulation of the active ingredient Levodopa and limits its conversion to dopamine at the peripheral level, with a consequent increase of efficacy at the central level.

The control of the release rate of these two active ingredients is of great therapeutic interest but presents technical problems whenever it is desired to administer them in a single system at different release kinetics, because the two drugs have very different water solubilities (500 mg/ml for Levodopa methyl ester at 20°C; 2.5 mg/ml for Carbidopa at 37°C). Furthermore the treatments with Levodopa involve different dosage schemes depending on Parkinson's disease symptoms. In some cases to rapidly treat tremors or lack of attention due to low blood levels of drug a rapid onset of effect is needed, whereas in other situations, maintaining the effective plasma level of Levodopa for a long time is necessary. This dual need, concerning the same active ingredient, requires the availability of pharmaceutical preparations which release the drug at different rates. The problem of controlled release is therefore particularly felt in the case of formulations of Levodopa and Carbidopa for the treatment of Parkinson's disease, as is clearly evidenced for example in the article published in Neurology,42 (1- Suppl.1), January 1992. In this article it is emphasized that the Levodopa controlled release formulations available on the market have a latency time in their activity that is commonly eliminated by administering an immediate release dose, together with the controlled release dose. It is therefore of extreme interest, for the patient's compliance, to dispose of a modified release formulation which reproduces the combined profile of a controlled release with an immediate release formulation.

The three-layer monolithic systems of the invention reproduce, through the administration of a single unit dose, the double and contemporary administration of a controlled release and an immediate release formulation. According to a preferred embodiment, the invention provides three-layer monolithic systems containing the two active ingredients, Levodopa methyl ester and Carbidopa, in the form of tablets obtained by compression of granular mixtures. Each mixture has a composition characterized by different release mechanisms, and is contained in a single layer of a multi-layer system, in order to give a monolithic system with combined release kinetics. The total dose of drug to administer is distributed in the three-layers in different ways according to the release mechanism of each layer, as a function of the therapeutic objectives and according to the chemical and physical properties of the drug to be released.

The disintegrating layer is based on an excipient which can quickly disintegrate on contact with water, mixed with a portion of the active ingredients and other excipients which favour the preparation of the granulate and the compression of the mixture. In particular, the disintegrating substances are selected from the so called super-disintegrants which are, in general, polymeric substances belonging to the class of crosslinked carboxymethyl cellulose derivatives, carboxymethylstarch, crosslinked polyvinylpyrrolidone and potassium methacrylate divinylbenzene. Disintegrants belonging to the class of starches or cyclodextrins and derivatives can also be useful. The super-disintegrants may be present in a percentage of 3% to 10% by weight, and the disintegrants of 3% to 30%.

The erodible layer is based on excipients belonging to the class of substances of lipidic nature and/or of soluble polymers, which impart to the layer the properties of being subject to a gradual erosion. Such adjuvants are, in the case of lipidic substances, semi-synthetic glycerides and, in the case of soluble polymers, polivinylpirrolidone, cellulose derivatives, polyvinylalcohols, cyclodextrins and their derivatives. Of particular importance for the functioning of the erodible layer is the process of preparation of the granular mixture which undergoes compression to form the erodible layer of the monolithic system. The best results have been obtained by granulating a mixture containing a portion of the two drugs, the semi-synthetic glycerides, a half of the quantity of lactose and the other excipients with an alcoholic solution of polyvinylpyrrolidone. To achieve a compression of the system and the optimum release of the active ingredient, this granulate is mixed with a second granulate obtained with the remaining quantity of lactose granulated with an alcoholic solution of polyvinylpyrrolidone. The content of lipidic substances in the erodible layer can vary between 1% and 5% by weight

The swelling layer is based on a polymer which swells in water, belonging to the class of cellulose derivatives like hydroxypropylmethylcellulose with a molecular weight (Mw) of between 10,000 and 1,000,000 Da, carboxymethylcellulose of medium to high viscosity and polyethylene oxide with a molecular weight of between 100,000 and 8,000,000. Of particular importance for the functioning of the swelling layer is the preparation of the granular mixture of the active ingredient with the swelling polymer and other excipients for granulation and compression, which must be carried out using a solution of hydroxypropylmethyl cellulose phthalate in acetone and water (95:5), in which triacetin is dissolved as a plasticizing agent in amount equal to 20% of the binding polymer.

The three-layer monolithic system in the form of a tablet as per the invention is prepared by compression in a rotary press provided with three loading hoppers, each filled with one of the three granulate corresponding to the three-layers with different release mechanism. The press deposits the proper amount of granulate, one on top of the other, according to the weight of each layer, in a predetermined sequence, in order to obtain a trilayer monolythic system with the desired release profile. The powder bed constituted of the three different granular mixtures, is compressed at a pressure which enables to produce a coherent monolithic system with suitable mechanical strength. The multilayer monolithic system of the invention can be provided with a break-line to allow the use of halved tablets.

The preferred shape of the monolithic system of the invention is that of a cylinder, but it may have different geometrical shapes such as oval, ovoidal, ellipsoidal or asymmetrical shape.

### Dissolution test

To evaluate the *in vitro* release of the active ingredients from the three-layer monolithic system the dissolution apparatus type 2, (described in USP XXII <711>, pg 1578-1579), was used with a paddle rotation speed of 100 rpm. A square screen (I = 33 mm; openings 1.25 mm) was placed on the bottom of the dissolution vessel to prevent the multilayer tablets from sticking to it. During the first hour of dissolution, artificial gastric fluid without enzymes as described in USP XXII was used as dissolution medium. In the following four hours, the dissolution fluid was substituted by a pH 5.5 phosphate buffer, described in British Pharmacopoeia 1993. All the dissolution fluids were thermostatically controlled at 37°C. The release of the active ingredients was determined by High Pressure Liquid Chromatography (HPLC), taking samples at pre-established intervals corresponding to: 30, 60, 120, 180, 240, and 300 minutes. The results obtained are shown in Tables 1, 2 and 3.

From these results, it appears that the interaction between the release mechanisms of the three-layers of the monolithic system, in the case of RED system wherein the erodible layer is the central layer (Table 1), gives a fast initial release of the active ingredients in the first 60 minutes of dissolution, followed by a slower and almost linear release, in particular of LDME, Levodopa methyl ester, which is completed within 300 minutes from the beginning of the dissolution test In the case of the DRE system, in which the swelling layer is the central layer (Table 2), an anomalous Fickian kinetic is observed for both active ingredients according to N.A. Peppas's Analysis of Fickian and non-Fickian drug release from polymers, Pharm. Acta, Helv. 1985, 60, 4110-4111 during all the release process, without evidence of biphasic release. Lastly, in the case of RDE system, wherein the disintegrating layer is the central layer (Table 3), a linear release is observed in the first 180 minutes of dissolution.

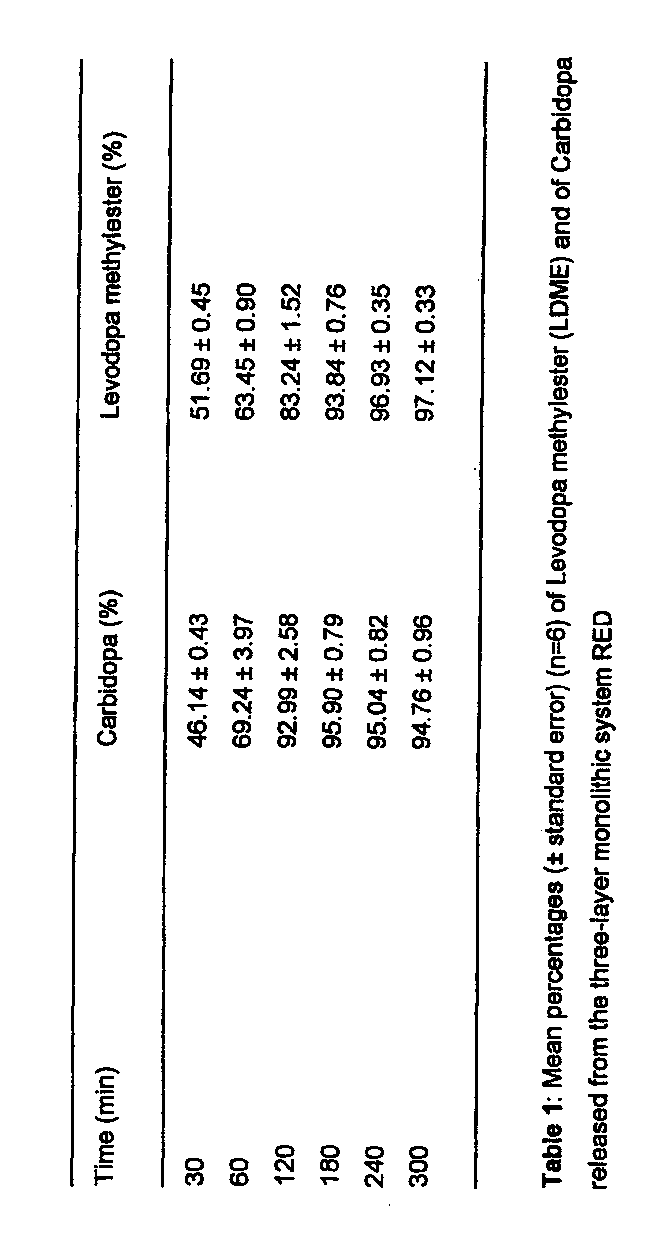

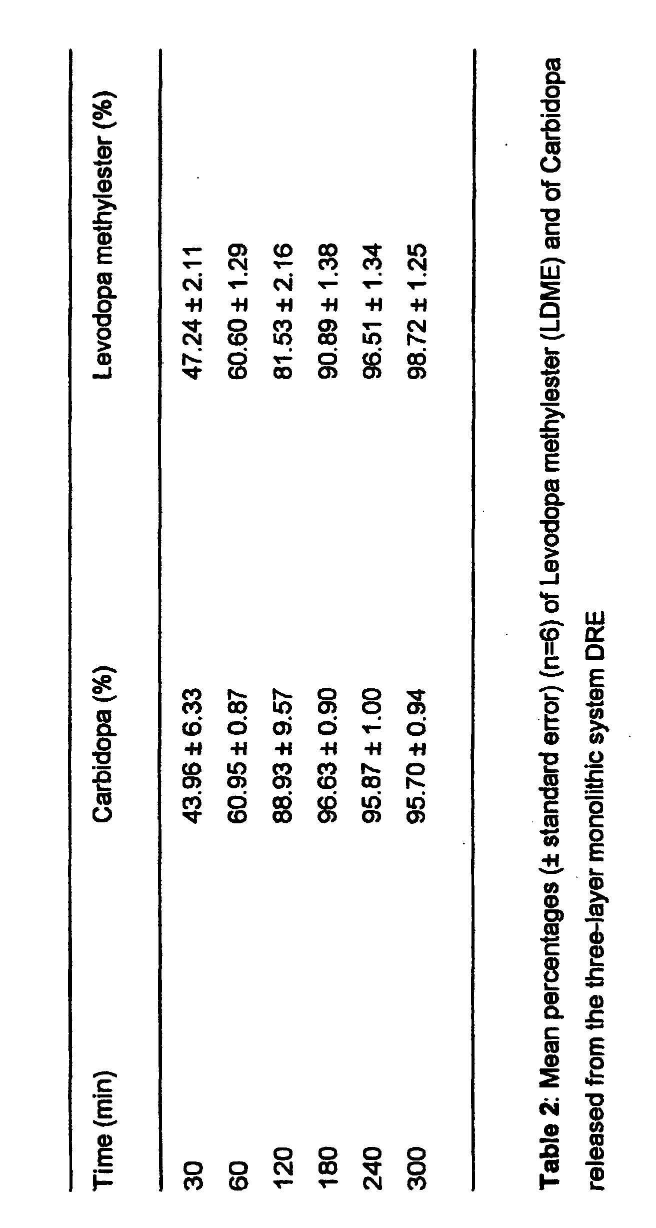

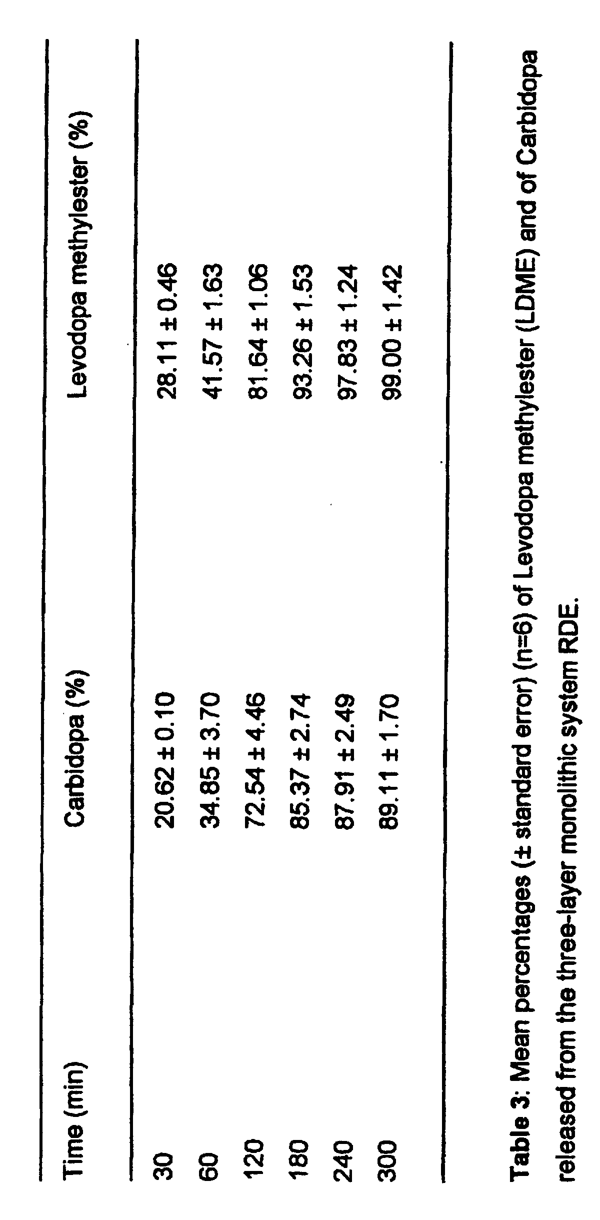

Tables 4, 5 and 6 show the percentages of drugs released by the single layers which represent the release kinetics characteristic of the release mechanism of each layer. Table 4 shows the fast release of the two active ingredients from the layer based on the disintegration; Table 5 shows the almost linear release kinetic of the two active ingredients from the layer based on the erosion mechanism; and lastly, Table 6 shows the typical kinetic known as anomalous-Fickian, referred to the drug contained in the swelling layer.

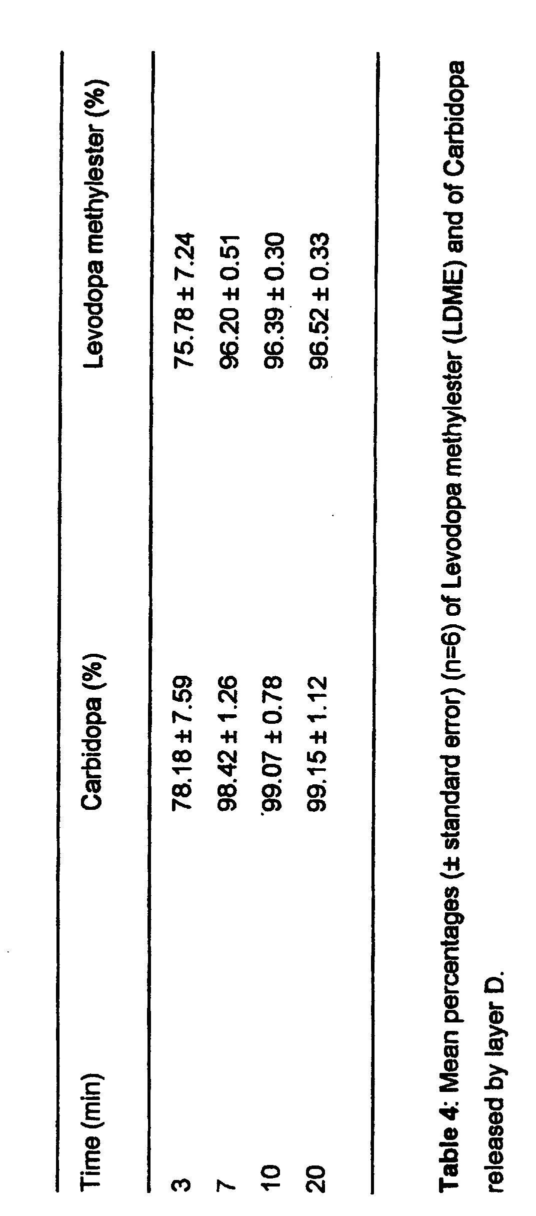

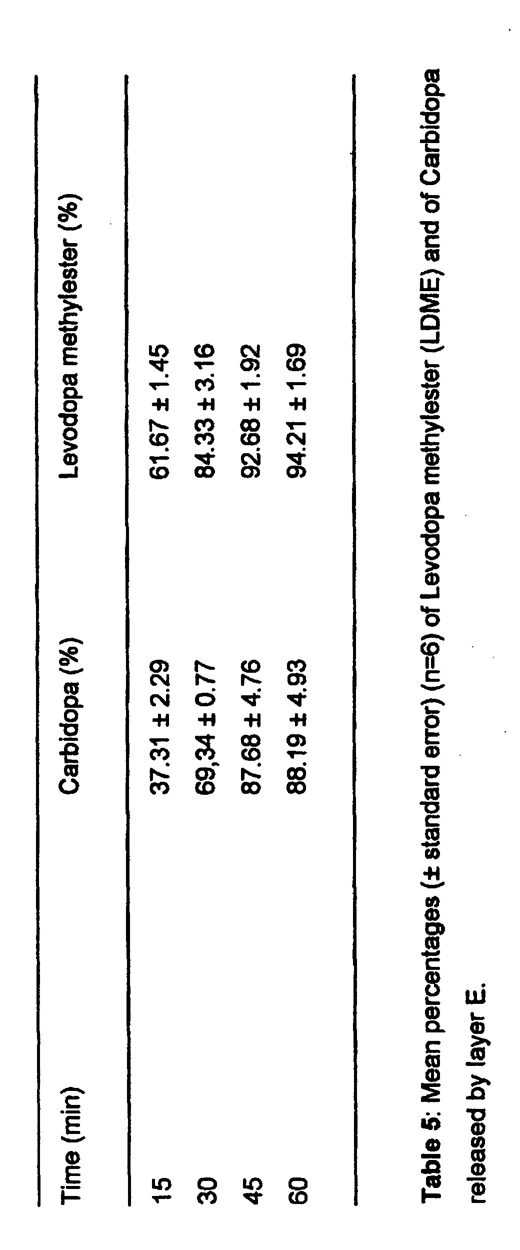

**Table 6**

| : Mean percentages (± standard error) (n=6) of Levodopa methylester (LDME) released from layer R | |
|---|---|
| Time (min) | Levodopa methylester (%) |
| 30 | 27.66 ±0.70 |
| 45 | 41.59 ±0.84 |
| 60 | 59.41 ±1.19 |
| 120 | 84.20 ± 1.36 |
| 180 | 95.63 ± 1.37 |
| 240 | 99.96 ± 1.05 |
| 300 | 100.86 ± 0.54 |

A comparison between the release profile of the individual layers with the release obtained with the three-layer system in examples 1, 2 and 3, clearly demonstrates the interaction produced by the combination of the three-layers in a monolithic system. The comparison demonstrates that three different mechanisms able to release the drug from mono-layer matrices according to different kinetics, if combined in a three-layer monolithic system, by the effect of their interaction, give rise to a whole release kinetics which are totally different from those of the individual layers, said release kinetics furthermore differing according to the relative positions of the layers in the three-layer monolithic system.

The interaction between the release mechanisms of the three layers in the monolithic system is again more evident in Table 7, where the total percentage of the active ingredients released from the three monolayer systems placed together in the dissolution medium are reported. In this experiment, each monolayer system has the same composition of the single layers constituting the monolithic systems of the invention and has been prepared by individually compressing the granular mixture used for the preparation of the disintegrating, erodible and swelling layers of the three-layer monolithic system.

The same release profiles as described in Tables 1, 2 and 3 have been obtained with compositions of three-layer monolithic system RED, DRE and RDE in which the amount of the components (both active ingredients and excipients) was reduced by a third.

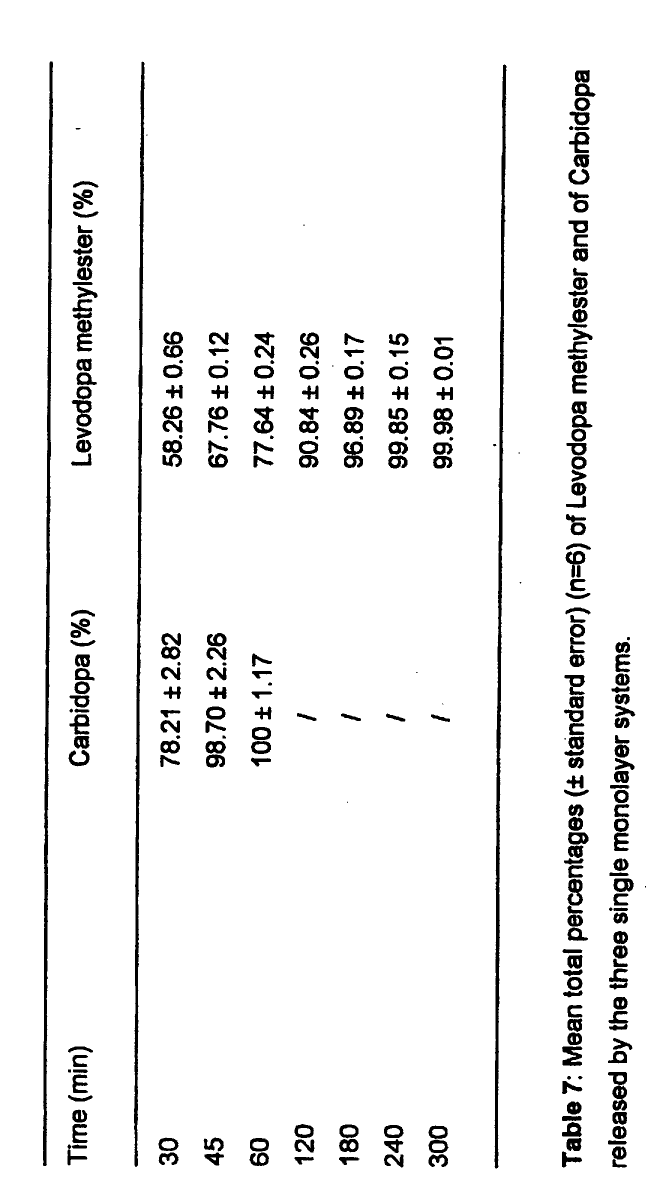

The *in vitro* release of modified release formulations does not always correlate with *in-vivo* behaviour. The lack of correlation between *in-vitro* and *in-vivo* results is generally due to limitations in the technology of the release systems production responsible for the release mechanisms of the active ingredient which occur in the *in-vitro* tests. In-vitro profiles, are not always representative of the in-vivo kinetics of the modified release formulations.

In order to verify the correspondence between the *in-vivo* and *in-vitro* behaviour of the three-layer monolithic system, a bioavailability study, in healthy volunteers (n=6) in accordance with a randomized cross-over design, has been carried out. In each of the study periods, which were separated by a seven-day wash out period, each volunteer received, on empty stomach, a single doses of the formulations. The experimental formulations were selected, on the basis of *in-vitro* release profiles, to be more interesting for therapeutic applications. The RDE and DRE systems having layers of equivalent composition but disposed in a different order, have been tested.

As reference treatment, the extemporary combination commonly used in therapy was used. A dose of the commercial product Sinemet®CR: (Levodopa 200 mg, Carbidopa anhydrous 50 mg) (controlled release form) together with a half dose of the immediate release product Sinemet®: (Levodopa 250 mg, Carbidopa anhydrous 25 mg) were administered. As this dosage scheme is considered to be the most suitable in avoiding latency effects which can occur with the controlled release formulations presently available, it is of great interest to see if, among the different kinetics obtained modifying the relative layers position in the three-layer monolithic system, there will be one which reproduces the pharmacokinetic profile of the reference administration whose use is however rather complex for the patient.

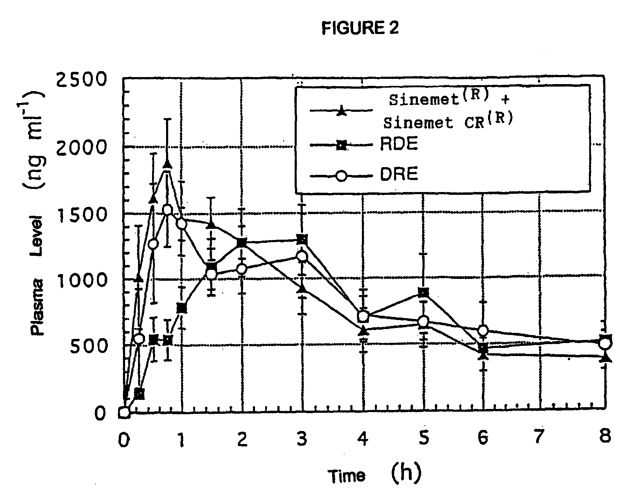

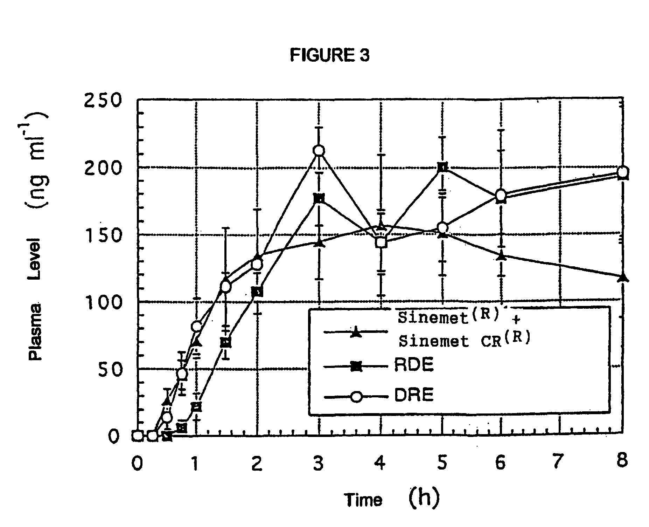

Blood samples have been taken in the 8 hours period following the treatment. Plasma levels of Levodopa and Carbidopa, were determined according to a validated HPLC/EC method. Levodopa and Carbidopa plasma levels are shown in Figures 2 and 3. In Tables 8 and 9, Levodopa and Carbidopa main pharmacokinetic parameters, obtained following the administration of the test formulations and the reference treatment, are reported.

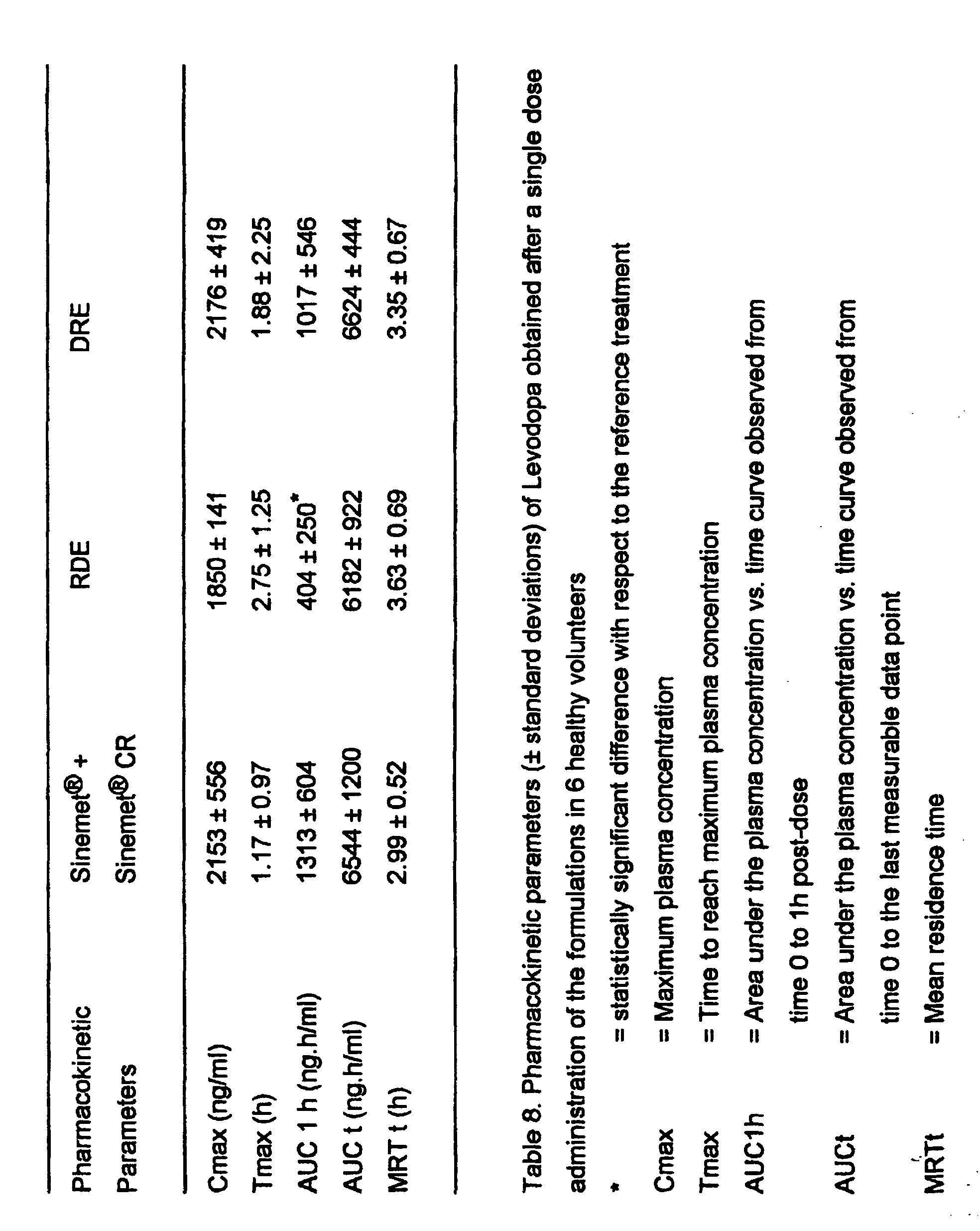

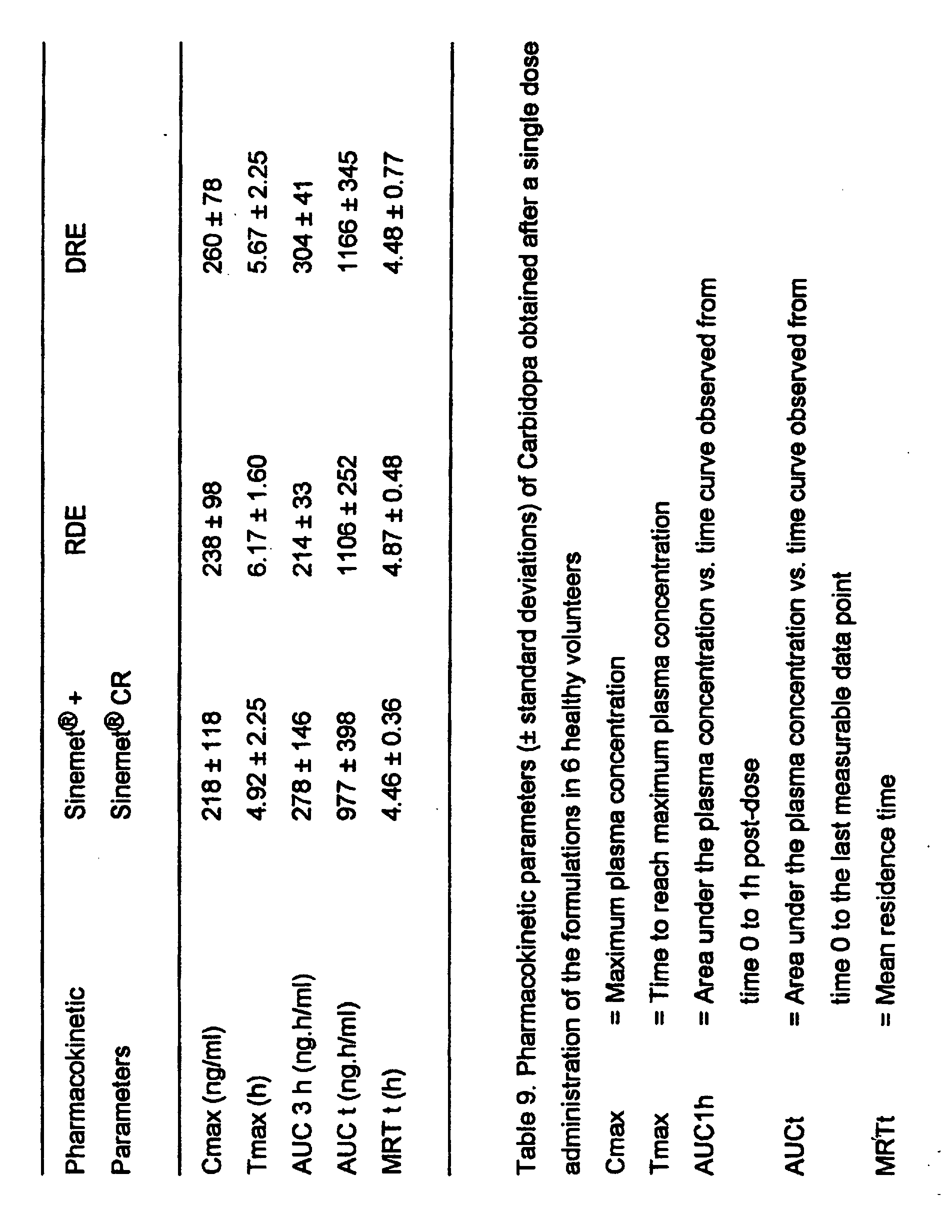

*In vivo*, the DRE system differs from the RDE monolithic system, in the bioavailability of Levodopa in the first hour after administration (AUC1h), due to the fast release of Levodopa methylester. This result points out the importance of the outside position of the erodible and disintegrating immediate release layers in the three-layer monolith. In fact, these layers release 100% of the inhibitor dose and 47% of the Levodopa methylester dose. The slow release swelling layer which contains the remaining quantity of Levodopa methylester (53% of the dose), determines the maintenance of the Levodopa plasma levels in the following 4 to 5 hours. The total bioavailability of Levodopa and Carbidopa (AUCt) is comparable in the two monolithic systems.

The results of the study demonstrate that the three-layer monolithic system -DRE- provides a Levodopa plasma profile which is not significantly different from that obtained with the extemporary combination of the two Levodopa commercial formulations. Furthermore, it has been verified that a type B correlation exists between the *in-vitro* dissolution data of Levodopa methylester and the *in-vivo* data obtained for Levodopa. The results obtained also demonstrate that the interaction of the release mechanism, due to the difference in the position of the layers in a three-layer monolith, is an *in-vivo* reproducible effect with interesting therapeutic applications of the release system.

This permits to deal Parkinson's disease with therapeutic instruments adequate to the different prescription requirements.

### Brief description of the Figures:

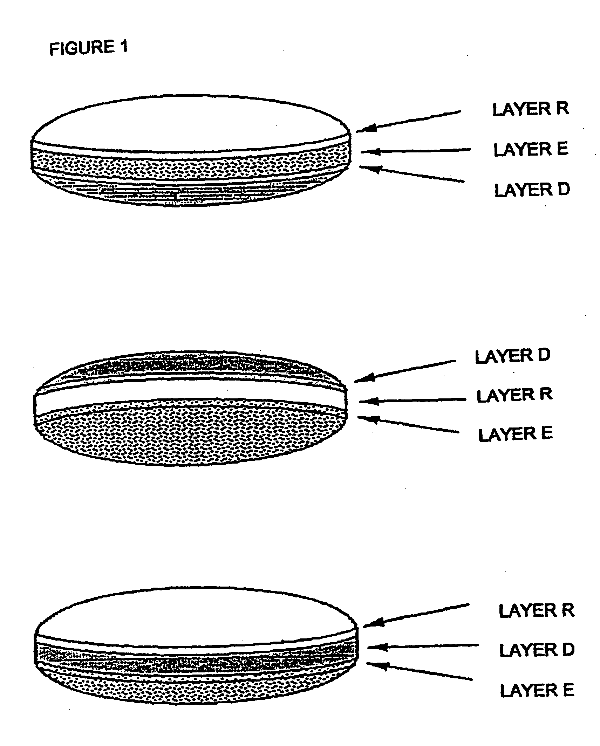

Figure 1: three-layer monolithic systems obtained by changing the relative position of the disintegrating layer D, the erodible layer E and the swelling layer R. a) monolithic system RED; b) monolithic system DRE, c) monolithic system RDE.
Figure 2: Plasma levels of Levodopa obtained after administration of Sinemet® + Sinemet®CR, and of the three-layer monolithic systems DRE and RDE. The bars represent the mean standard error (n=6).
Figure 3: Plasma levels of Carbidopa obtained after administration of Sinemet® + Sinemet®CR and of the three-layer monolithic systems DRE and RDE. The bars represent the mean standard error (n=6).

The following examples illustrate the invention in greater detail.

### Example 1: Three-layer monolithic system RED.

Preparation of the three-layer monolithic system in which the layer order is as follows: swellable layer R; erodible layer E; disintegrating layer D.

### a) Preparation of the granular mixture for the swelling laver R

100 g of the mixture contain (the amount in mg of each component per unit dose is indicated in brackets):

| | |
|---|---|
| Levodopa methyl hydrochloride | 70.80 g (201.00 mg) |
| Hydroxypropylmethylcellulose (Methocel®K15M, Colrcon, Orpington, UK) | 20.10 g (57.00 mg) |
| Eudralack® red (Rohm Pharma, Darmstadt, Germany) | 0.01 g (0.03 mg) |
| Hydroxypropylmethylcellulose phthalate (HPMCP 50, Eastman, TN, USA) | 3.29 g (9.47 mg) |
| Triacetin (Eastman, TN, USA) | 0.70 g (2.00 mg) |
| Talc | 4.10 g (11.60 mg) |
| Magnesium stearate | 1.00 g (2.90 mg) |

Weigh Levodopa methylester, Carbidopa, Eudralack® red and hydroxypropylmethylcellulose on a technical balance. Mix the powders in a mortar for 15 minutes and wet the mixture with a solution of hydroxypropylmethylcellulose phthalate and triacetin in 50 ml of an acetone/water mixture (95:5) until a uniform paste is obtained. Sieve the paste through a 710 µm screen, oven dry at a temperature of 30°C, for 12 hours and size the dried granulate using the same screen. Mix the granulate with talc and magnesium stearate in a Turbula® mixer for 15 minutes. The granular mixture is now ready for compression.

### b) Preparation of the granular mixture for the erodible layer E

100 g of the mixture contain (the amount in mg of each component per unit dose is indicated in brackets):

| | |
|---|---|
| Levodopa methyl hydrochloride | 21.6 g (50.20 mg) |
| Carbidopa hydrate | 23.0 g (54.00 mg) |
| Potassium metabisulfite | 0.5 g (1.20 mg) |
| Blue lake (E 132) (Rohm Pharma, Darmstadt, Germany) | 0.1 g (0.20 mg) |
| Glyceryl palmitostearate (Precirol® Ato) | 5.0 g (11.60 mg) |
| Lactose | 43.0 g (100.10 mg) |
| Polyvinylpyrrolidone (Plasdone® K29-32, GAF Corp., Wayne, NY, USA) | 1.8 g (4.10 mg) |
| Talc | 4.0 g (9.30 mg) |
| Magnesium stearate | 1.0 g (2.30 mg) |

Weigh the components, Levodopa methylester, Carbidopa, potassium bisulfite, blue lake, Precirol® and a half portion of lactose on a technical balance. Mix the powders in a mortar for 15 minutes, wet the mixture with a solution of 1.3 g of polyvinylpyrrolidone in 17 ml of 95% ethanol and sieve the paste through a 710 µm screen. Then granulate the remaining 21.35 g of lactose with a solution of 0.4 g of polyvinylpyrrolidone in 4 ml of 95% ethanol. Oven dry the granulates at a temperature of 30°C for 12 hours and size the dried granulates using a 710 µm screen. Mix the two granulates with talc and magnesium stearate in a Turbula® mixer for 15 minutes. The granular mixture is now ready for compression.

### c) Preparation of the granular mixture for the disintegrating layer D

100 g of the mixture contain (the amount in mg of each component per unit dose is indicated in brackets):

| | |
|---|---|
| Levodopa methyl hydrochloride | 65.1 g (125.6 mg) |
| Carbidopa hydrate | 14.0 g (27.0 mg) |
| Eudralack® yellow | 0.5 g (1.00 mg) |
| Polyvinylpyrrolidone (Plasdone® K29-32,GAF Corp., Wayne, NY, USA) | 1.0 g (2.0 mg) |
| Microcrystalline cellulose(Avicel® PH102, FMC Corp, PA, USA) | 10.2 g (19.6 mg) |
| Talc | 4.1 g (7.9 mg) |
| Magnesium stearate | 1.0 g (2.0 mg) |
| Croscarmellose sodium (Ac-Di-Sol®, FMC Corp, PA, USA) | 4.1 g (7.9 mg) |

Weigh the components, Levodopa methylester, Carbidopa, Eudralack® yellow and microcrystalline cellulose. Mix the powders in a mortar for 15 minutes and wet the mixture with a solution of 1 g of polyvinylpyrrolidone in 10 ml of 95% ethanol. Sieve the paste through a 710 µm screen, oven dry the granulate at a temperature of 30°C for 12 hours and size the dried granulate using the same screen. Mix the granulate with talc, magnesium stearate and croscarmellose sodium in a Turbula® mixer for 15 minutes. The granular mixture is now ready for compression.

### d) Preparation of the three-layer monolithic system

The three-layer monolithic systems of the example have been obtained using a single punch tableting machine (model EKO, Korsch, Berlin, Germany) equipped with 12 mm diameter round concave punches, by the following procedure: weigh the powder mixtures which constitute each layer in the amounts of: 284 mg for layer R, 233 mg for layer E and

193 mg for layer D. Place the weighed mixtures in the die in the sequence RED and after having carried out a light pre-compression after each placing to reduce the volume of the powder bed, press at a pressure between 2,000 and 4,000 Kg/cm² to obtain a three-layer monolithic system with good mechanical strength, having a theoretical weight of 710 mg and a thickness of about 6.3 mm.

### Example 2: Three-layer monolithic system DRE

Preparation of a 3 layer monolithic system in which the layer sequence is as follows: disintegrating layer D; swelling layer R; erodible layer E.

### a) Preparation of the granular mixture for disintegrating layer D.

The mixture is prepared as described in point c) of example 1.

### b) Preparation of the granular mixture for swelling layer R.

The mixture is prepared as described in point a) of example 1.

### c) Preparation of the granular mixture for erodible layer E.

The mixture is prepared as described in point b) of example 1.

### d) Preparation of the three-layer monolithic system

The three-layer monolithic systems of the example have been obtained using a single punch tableting machine (model EKO, Korsch, Berlin, Germany) equipped with 12 mm diameter round concave punches by the following procedure:
weigh the powder mixtures which constitute each layer, in the amounts of: 193 mg for layer D, 284 mg for layer R and 233 mg for layer E. Place the weighed mixture in the die in the sequence DRE and after having carried out a light pre-compression after each placing to reduce the volume of the powder bed, press at a pressure between 2,000 and 4,000 Kg/cm² to obtain a three-layer monolithic system with good mechanical strength, having a theoretical weight of 710 mg and a thickness of about 6.3 mm.

### Example 3: Three-layer monolithic system RDE

Preparation of a three-layer monolithic system in which the layer sequence is as follows: swelling layer R; disintegrating layer D; erodible layer E.

### a) Preparation of the granular mixture for swelling layer R.

The mixture is prepared as described in point a) of example 1.

### b) Preparation of the granular mixture for disintegrating layer D.

The mixture is prepared as described in point c) of example 1.

### c) Preparation of the granular mixture for erodible layer E.

The mixture is prepared as described in point b) of example 1.

### d) Preparation of the three-layer monolithic system

The three-layer monolithic systems of the example have been obtained using a single punch tableting machine (model EKO, Korsch, Berlin, Germany) equipped with 12 mm diameter round concave punches, by the following procedure: weigh the powder mixtures which constitute each layer in the amounts of: 284 mg for layer R, 193 mg for layer D and 233 mg for layer E. Place the weighed mixtures in the die in the sequence RDE and after having carried out a light pre-compression after each placing to reduce the volume of the powder bed, press at a pressure between 2,000 and 4,000 Kg/cm² to obtain a three-layer monolithic system with good mechanical strength, having a theoretical weight of 710 mg and a thickness of about 6.3 mm.

## Claims

1. A three-layer oral controlled release tablet prepared by compression of the granulates of the different layers consisting of a disintegrating layer, an erodible layer and a swelling layer, two external and one intermediate, wherein the swelling layer contains Levodopa methyl ester and the erodible and disintegrating layers contain Levodopa methyl ester and Carbidopa.

2. A tablet according to claim 1, wherein the swelling layer is interposed between the erodible layer and the disintegrating layer.

3. A tablet according to claim 1, wherein the erodible layer is interposed between the swelling layer and the disintegrating layer.

4. A tablet according to claims 1 to 3, wherein the excipients of the disintegrating layer are selected from the group consisting of cross-linked carboxymethylcellulose, carboxymethylstarch, cross-linked polyvinylpyrrolidone, potassium methacrylate divinyl benzene, starches, cyclodextrins and derivatives.

5. A tablet according to claim 4 wherein the excipients are present in amounts of between 3% and 10% by weight.

6. A tablet according to claims 1 to 5 wherein the excipients of the erodible layer are selected from the group comprising semi synthetic glycerides, polyvinylpyrrolidone, cellulose derivatives, polyvinylalcohol, cyclodextrins and derivatives.

7. A tablet according to claim 6 wherein the semi-synthetic glycerides are present in amounts of between 3% and 15% by weight

8. A tablet according to claims 1 to 7 wherein the excipients of the swelling layer are selected from the group consisting of hydroxypropylmethylcellulose, carboxymethylcellulose and polyethylene oxide.

9. A tablet according to claim 8 wherein the swelling layer further contains triacetin as a plasticizing agent.

10. Process for preparing the tablet of claims 1-9 comprising the compression of granulates of the different layers.

11. Tablet according to any of claims 1-9 for use in the therapy of Parkinson's disease.

## Patentansprüche

1. Aus drei Schichten bestehende oral kontrolliert verabreichte Tablette, welche durch die Kompression der Granulate der drei unterschiedlichen Schichten hergestellt wird, welche aus einer sich auflösenden Schicht, einer erodierenden Schicht und einer quellenden Schicht bestehen, von denen zwei außen und eine dazwischenliegend angeordnet ist, wobei die quellende Schicht Levodopa Methylester enthält, während die erodierbare Schicht und die sich auflösende Schicht Levodopa Methylester und Carbidopa enthalten.

2. Tablette nach Anspruch 1,
**dadurch gekennzeichnet, dass**
die quellende Schicht zwischen der erodierbaren Schicht und der sich auflösenden Schicht angeordnet ist.

3. Tablette nach Anspruch 1,
**dadurch gekennzeichnet, dass**
die erodierbare Schicht zwischen der quellenden Schicht und der sich auflösenden Schicht angeordnet ist.

4. Tablette nach einem der Ansprüche 1 bis 3,
**dadurch gekennzeichnet, dass**
die Arzneistoffträger der sich auflösenden Schicht aus der Gruppe ausgewählt werden, die vernetzte Carboxymethylcellulose, Carboxymethylstärke, vernetztes Polyvinylpyrrolidon, Kaliummethylacrylatdivinylbenzen, Stärken, Cyclodextrinen und ihre Derivaten umfasst.

5. Tablette nach Anspruch 4,
**dadurch gekennzeichnet, dass**
die Arzneistoffträger in einer Menge zwischen 3 Gew.-% und 10 Gew.-% vorhanden sind.

6. Tablette nach einem der Ansprüche 1 bis 5,
**dadurch gekennzeichnet, dass**
die Arzneistoffträger der erodierbaren Schicht aus der Gruppe ausgewählt werden, welche halbsynthetische Glyceride, Polyvinylpyrrolidon, Cellulosederivate, Polyvinylalkohol, Cyclodextrine und deren Derivate umfasst.

7. Tablette nach Anspruch 6,
**dadurch gekennzeichnet, dass**
die halbsynthetischen Glyceride in einer Menge zwischen 3 Gew.-% und 15 Gew.-% vorhanden sind.

8. Tablette nach einem der Ansprüche 1 bis 7,
**dadurch gekennzeichnet, dass**
die Arzneistoffträger der quellenden Schicht aus der Gruppe ausgewählt werden, welche Hydroxypropylmethylcellulose, Carboxymethylcellulose und Polyethylenoxid umfasst.

9. Tablette nach Anspruch 8,
**dadurch gekennzeichnet, dass**
die quellende Schicht außerdem Triacetin als Weichmacheragens enthält.

10. Verfahren für die Herstellung einer Tablette nach einem der Ansprüche 1 bis 9,
**dadurch gekennzeichnet, dass**
die Tablette durch die Kompression der Granulate der verschiedenen Schichten hergestellt wird.

11. Tablette nach einem der Ansprüche 1 bis 9,
**dadurch gekennzeichnet, dass**
sie für die Behandlung der Parkinson'schen Krankheit eingesetzt wird.

## Revendications

1. Comprimé oral à libération régulée à trois couches préparé par compression des granulats des différentes couches, constitué par une couche de désintégration, une couche érodable et une couche gonflante, deux externes et une intermédiaire, dans lequel la couche gonflante contient de l'ester méthylique de Lévodopa et les couches érodable et de désintégration contiennent de l'ester méthylique de Lévodopa et du Carbidopa.

2. Comprimé selon la revendication 1, dans lequel la couche gonflante est intercalée entre la couche érodable et la couche de désintégration.

3. Comprimé selon la revendication 1, dans lequel la couche érodable est intercalée entre la couche gonflante et la couche de désintégration.

4. Comprimé selon les revendications 1 à 3, dans lequel les excipients de la couche de désintégration sont choisis dans le groupe constitué par la carboxyméthylcellulose réticulée, l'amidon carboxyméthylé, la polyvinylpyrrolidone réticulée, le méthacrylate de potassium divinylbenzène, les amidons, les cyclodextrines et les dérivés.

5. Comprimé selon la revendication 4, dans lequel les excipients sont présents en des proportions entre 3 % et 10 % en poids.

6. Comprimé selon les revendications 1 à 5, dans lequel les excipients de la couche érodable sont choisis dans le groupe comprenant les glycérides semi-synthétiques, la polyvinylpyrrolidone, les dérivés de cellulose, l'alcool polyvinylique, les cyclodextrines et les dérivés.

7. Comprimé selon la revendication 6, dans lequel les glycérides semi-synthétiques sont présents en des quantités entre 3 % et 15 % en poids.

8. Comprimé selon les revendications 1 à 7, dans lequel les excipients de la couche gonflante sont choisis dans le groupe constitué par l'hydroxypropylméthyl-cellulose, la carboxyméthylcellulose et l'oxyde de polyéthylène.

9. Comprimé selon la revendication 8, dans lequel la couche gonflante contient en outre de la triacétine en tant qu'agent plastifiant.

10. Procédé pour préparer le comprimé selon les revendications 1-9, comprenant la compression de granulats des différentes couches.

11. Comprimé selon l'une quelconque des revendications 1-9 pour l'utilisation dans la thérapie de la maladie de Parkinson.
